# EUROPEAN PATENT SPECIFICATION

(11) **EP 1 146 964 B1**
(45) Date of publication and mention of the grant of the patent: **19.03.2003**
(21) Application number: 99972947.8
(22) Date of filing: 01.12.1999
(51) Int. Cl.: B02C 19/06

(54) **MILLING PROCESS FOR THE PRODUCTION OF FINELY MILLED MEDICINAL SUBSTANCES**
ZERKLEINERUNGSVERFAHREN ZUR HERSTELLUNG VON FEINGEMAHLENEN MEDIZINISCHEN SUBSTANZEN
PROCEDE DE BROYAGE AUX FINS DE LA PRODUCTION DE SUBSTANCES MEDICINALES FINEMENT BROYEES

(30) Priority: 01.12.1998 GB 9826286
(43) Date of publication of application: 24.10.2001
(60) Divisional application: 02079497.0
(73) Proprietor: Aventis Pharma Limited, West Malling, Kent ME19 4AH (GB)
(72) Inventor: AUTHELIN, Jean-René, Rhône-Poulenc Rorer S.A., F-94403 Cedex Vitry-Sur-Seine (FR); HOSEK, Patrick, Rhône-Poulenc Rorer S.A., F-94403 Cedex Vitry-Sur-Seine (FR)
(74) Representative: Jones, Stephen Anthony
(86) International application number: PCT/GB99/04047
(87) International publication number: WO 00/032313

(56) References cited:
- WO-A-97/32668
- WO-A-98/31352
- GB-A- 1 481 304
- US-A- 4 767 612
- US-A- 5 747 002

## Description

The present invention relates to a process for the production of finely milled medicinal substances intended for use as inhalation medicaments.

Inhalation medicaments must have a fine particle size in order to penetrate deep into the lungs where they can be absorbed. Typically particles less than 10 microns in size are required. Such fine particles are normally prepared by milling the material to be inhaled. It is well known that the intensive milling required to produce these fine particle sizes can produce profound changes in the crystal structure of the material being milled. The exact changes are governed by the nature of the starting material but commonly freshly milled powders have a greatly increased content of amorphous phase. This initially forms on the surface of the particles but can constitute a large proportion of the total weight of the powder.

Changes in crystal structure, including increase in amorphous content. can cause a number of problems. The particles tend to stick together, making the freshly milled powder extremely cohesive. With time, often under the influence of ambient moisture, the surface phase tends to revert to its more stable original phase. This can cause the particles to be welded together. Furthermore, the crystal form of a pharmaceutical substance can have a significant effect on its potency, as discussed by J.I. Wells in Pharmaceutical Preformulation: The Physiochemical Properties of Drug Substances, John Wiley & Sons, New York (1988). We have now found that by careful control of the milling conditions used we can achieve the required particle size for an inhalation medicament without generating amorphous phases on the surface of the powder.

US5562923 describes a method for producing finely milled highly crystalline medicinal substances intended for use as inhalation medicaments by drying the milled medicament, treating with a non aqueous solvent and then drying. US 5637620 uses a different Method; the Milled Medicament is conditioned under controlled conditions of temperature and humidity before being dried.

In a fluid energy mill the material to be milled is entrained in an airstream and the particles caused to collide with one another by turbulence in the air stream. However, the energy input to the powder surface tends to produce a phase change to an amorphous state. One possible solution to this problem would be to mill at a reduced temperature. The material to be milled is likely to be more brittle and friable, resulting in a lower energy input to each powder particle. Also phase change reactions tend to proceed more slowly at lower temperatures. To be effective temperatures well below 0°C are required. One problem with this approach is that the milling fluids most commonly used, nitrogen and air, become less effective as their temperature drops. In particular the exit velocity of the gas from the milling nozzles becomes too low.

WO 97/32668 discloses an improved fluid energy mill comprising an insert having a leading edge and a trailing edge with an azimuthal angle of between 10° and 300°. GB 1481304 discloses the use of a milling fluid at reduced temperature to produce a powder. WO 98/31352 discloses a dry powder composition having a poured bulk density of from 0.28 to 0.38 g/ml. US 4767612 discloses a method of treating allergic rhinitis comprising the administration of an effective amount of the disclosed micronised medicament in suspension into the nasal cavity of the patient.

We have now found that this problem can be overcome by using helium as milling fluid. The process provides finely milled, highly crystalline material containing substantially no amorphous material. A surprising advantage is that build up of scale in the mill during milling is much reduced. Less scale is deposited and the scale which is deposited is less hard and easier to remove.

Therefore, according to the present invention there is provided a method for producing fine, highly crystalline material consisting of fluid energy milling of crystalline material using a milling fluid comprising helium, wherein the temperature of the milling fluid is between -30°C and -120°C.

Pure helium can be used or a mixture of helium and another gas. Thus, for example, nitrogen and/or air can be mixed with helium. Pure helium is preferred. Preferably the milling temperature falls within the range of -50 to -70°C.

The milling process may be applied to any crystalline material. However it may particularly be used to mill medicament powders, especially medicament powders intended for administration by inhalation. It is particularly advantageous when applied to soft powders which are difficult to mill to a fine uniform particle size.

The particle size of the product is controlled in the conventional manner by adjusting pressure and flow rate of the milling fluid and feed rate of the material to be milled. Any equipment conventionally used in combination with a fluid energy mill to help control product particle size distribution can also be used in conjunction with the claimed method. The reduced tendency to form scale is particularly advantageous when a classifier is used in conjunction with the mill.

We have also found that it is possible to produce extra fine powder by the method described above. Milled powders with a median particle size as low as 1 micron can be produced. The lower limit of powder median particle size which is produced by conventional fluid energy milling is around 2 to 3 micron.

The amount of amorphous material in a sample of milled powder can be assessed in a number of ways. Differential Scanning Calorimetry (DSC) will show the heat of crystallisation in a sample containing amorphous material. Alternatively the change in weight of a sample exposed to an atmosphere of controlled temperature and humidity can give a measure of the change in amorphous content. In both methods the apparatus is calibrated using samples of known crystalline content and the unknown sample measured by comparing the magnitude of the measurement for the unknown with the known samples.

Also, amorphous substances usually have a substantially higher specific surface area than the corresponding crystalline substance. Thus, when an powder with an appreciable amorphous content crystallises the specific surface area falls. When a powder produced by conventional milling with a substantial amorphous content is stored in contact with the atmosphere the amorphous material tends to crystallise over a period of time. Within a few days, or weeks at most, surface area falls to a substantially stable value.

Accordingly, in the context of the present invention a powder may be considered to have substantially no amorphous content if its specific surface area does not change substantially when stored in a container open to the atmosphere for a week or more. The change in surface area should preferably be no more than 20% of the initial value, more preferably no more than 10% and most preferably no more than 5%. Alternatively a powder would be considered to have substantially no amorphous content if the level immediately after milling as measured by weight change under controlled relative humidity or DSC is less than 5%, more preferably less than 2% and most preferably less than 1%.

Surface area can be measured by gas absorption using the Brunauer-Emmet-Teller method or by air permeametry using the Blaine method. Results given here relate to the latter method which is described in the standard method of the l'Association Francaise de Normalisation (AFNOR) no P 15-442 March 1987.

Weight change under controlled relative humidity is measured using a DVS 1 dynamic vapour sorption apparatus. A small weighed sample is placed in a microbalance pan and held at constant temperature of 25°C and a relative humidity of 75%. Weight change is measured as a function of time over a period of at least 5 hours. The plot of weight v time shows a peak which- is proportional to the proportion of amorphous material present. The equipment is calibrated with samples of known amorphous content produced by mixing fully crystalline and fully amorphous materials.

DSC measurements were carried out using a Seiko RDC 220 system. The sample is weighed into the measuring pan and held at a temperature below the recrystallisation temperature for 30 minutes under a flow of dry nitrogen to remove any surface moisture. The sample was then heated at a constant rate of 20°C per minute. The exothermic peak due to recrystalisation is measured. As above the method is calibrated using samples of known amorphous content.

### Example

A two inch diameter pancake mill was used for the experiments. Helium is introduced to the circumference of the mill and powder to be milled is blown in through a venturi orifice also entering through the circumference of the milling chamber. Milled product, entrained in the milling fluid, exits through a central outlet. The temperature of the milling gas and/or the feed gas can be controlled.

The table below gives results obtained when milling triamcinolone acetonide (TAA) according to the present invention. The same feed was used in all cases and the starting material had a median particle size (d50) as measured by Malvern particle size analyser of around 25 micron. The gas used was helium or nitrogen in all cases.
Surface area was measured using the Blaine air permeability method. Where samples were stored for ageing trials the samples were kept in a 60% relative humidity atmosphere at 25°C.
Run 1 and Run 2 compare the effects of room temperature helium and nitrogen as milling gas. Helium gives a finer, higher surface area product but both products have a relatively high amorphous content.

Run 3 used nitrogen at -7°C as milling gas. Again a relatively high amorphous content was produced.

Run 4 and Run 5 used cold helium as the milling and carrier gas. The product had no detectable amorphous content and was also significantly finer than would be expected given the milling conditions

| | Run 1 | Run 2 | Run 3 | Run 4 | Run 5 |
|---|---|---|---|---|---|
| Feeding rate (kg/h) | 0.1 | 1 | 0.1 | 1 | 1 |
| Milling pressure (bar) | 4 | 5 | 7 | 5 | 5 |
| Feed gas pressure (bar) | 5 | 7 | 9 | 7 | 7 |
| Gas | Helium | Nitrogen | Nitrogen | Helium | Helium |
| Temperature (°C) | Room T | Room T | -7 | -65 | - 50 |
| mill size (inches) | 2 | 4 | 2 | 4 | 4 |
| | | | | | |
| Product Sw (m²/g) | 3.2 | 1.5 | 1.2 | 3.0 | 3.3 |
| Product Sw (m²/g) after one week | - | - | - | 2.9 | - |
| Product Sw (m²/g) after two weeks | - | - | - | - | 3.3 |
| | | | | | |
| Product d50 (µm) | - | - | - | 1.5 | 1.5 |
| | | | | | |
| Amorphous content (%) | 7.6 | 3.2 | 5.8 | n.d. | n.d. |
| n.d. = not detected | | | | | |

Product from Run 5 was tested in an Ultrahaler® device and the results compared with product milled in the conventional way. The Ultrahaler® is a dry powder inhaler whose basic operation is described in EP 407 028.

A compact was produced by compressing a mixture of 5% milled product with 95% lactose with a median particle size of 50 micrometer. The compact is loaded into the inhaler and doses cut off from it using a blade. Up to 200 doses can be obtained from each device. The important parameters are dose uniformity and the percentage respirable fraction of medicament produced in each dose.

For product produced by conventional means the mean respirable fraction produced was 44% and 83% of the doses cut were within 20% of their nominal weight. For product produced under the conditions of Run 5 the mean respirable fraction was 40% but the percentage of doses within 20% of nominal weight rose to 90%.

## Claims

1. A method for producing fine, highly crystalline material consisting of fluid energy milling of crystalline material using a milling fluid comprising helium, wherein the temperature of the milling fluid is between -30°C and -120°C.

2. A method according to claim 1 wherein the milling fluid consists of helium.

3. A method according to claim 1 or claim 2 wherein the temperature of the milling fluid is between -50°C and -70°C.

4. A method according to any one of claims 1-3 wherein the crystalline material comprises a medicament powder.

5. A method according to claim 4 wherein the crystalline material is triamcinolone acetonide.

6. A method according to any one of claims 1-5 wherein the product has an amorphous content of less than 5%.

7. A method according to claim 6 wherein the product has an amorphous content of less than 2%.

8. A method according to claim 7 wherein the product has an amorphous content of less than 1%.

9. A method according to any one of claims 1-8 wherein the product consists of a medicament powder in a form suitable for inhalation.

10. A method according to claim 9 wherein the product has a median particle size of less than 10 microns.

## Patentansprüche

1. Verfahren zum Herstellen von feinem hoch kristallinem Material, umfassend das Strahlmahlen von kristallinem Material mit einem Mahlfluid umfassend Helium, bei dem die Temperatur des Mahlfluids zwischen -30°C und -120°C beträgt.

2. Verfahren nach Anspruch 1, bei dem das Mahlfluid aus Helium besteht.

3. Verfahren nach Anspruch 1 oder Anspruch 2, bei dem die Temperatur des Mahlfluids zwischen -50°C und -70°C beträgt.

4. Verfahren nach einem der Ansprüche 1 bis 3, bei dem das kristalline Material ein Medikamentenpulver umfasst.

5. Verfahren nach Anspruch 4, bei dem das kristalline Material Triamcinolon-Acetonid ist.

6. Verfahren nach einem der Ansprüche 1 bis 5, bei dem das Produkt einen amorphen Gehalt von weniger als 5 % hat.

7. Verfahren nach Anspruch 6, bei dem das Produkt einen amorphen Gehalt von weniger als 2 % hat.

8. Verfahren nach Anspruch 7, bei dem das Produkt einen amorphen Gehalt von weniger als 1 % hat.

9. Verfahren nach einem der Ansprüche 1 bis 8, bei dem das Produkt aus einem Medikamentenpulver in einer für die Inhalation geeigneten Form besteht.

10. Verfahren nach Anspruch 9, bei dem das Produkt eine mittlere Partikelgröße von weniger als 10 Mikrometern hat.

## Revendications

1. Procédé destiné à produire une matière fine hautement cristalline comportant le broyage à jet fluide d'une matière cristalline en utilisant un jet fluide de broyage comprenant de l'hélium, dans lequel la température du jet fluide de broyage est située entre -30 et -120°C.

2. Procédé suivant la revendication 1, dans lequel le jet fluide de broyage se compose d'hélium.

3. Procédé suivant la revendication 1 ou suivant la revendication 2, dans lequel la température du jet fluide de broyage est située entre -50 et -70°C.

4. Procédé suivant l'une quelconque des revendications 1 à 3, dans lequel la matière cristalline comprend une poudre de médicament.

5. Procédé suivant la revendication 4, dans lequel la matière cristalline est de l'acétonide de triamcinolone.

6. Procédé suivant l'une quelconque des revendications 1 à 5, dans lequel la poudre a une teneur en matière amorphe inférieure à 5 %.

7. Procédé suivant la revendication 6, dans lequel le produit a une teneur en matière amorphe inférieure à 2 %.

8. Procédé suivant la revendication 7, dans lequel le produit a une teneur en matière amorphe inférieure à 1 %.

9. Procédé suivant l'une quelconque des revendications 1 à 8, dans lequel le produit se compose d'une poudre de médicament sous une forme appropriée pour l'inhalation.

10. Procédé suivant la revendication 9, dans lequel le produit a une taille de particule moyenne inférieure à 10 microns.
